# EUROPÄISCHE PATENTANMELDUNG

(11) **EP 4 848 309 A1**
(43) Veröffentlichungstag der Anmeldung: **02.12.2026**
(21) Anmeldenummer: 25179988.8
(22) Anmeldetag: 30.05.2025
(51) Int. Cl.: G16H 50/50, A61C 13/00, A61C 13/09

(54) **COMPUTERIMPLEMENTIERTES VERFAHREN ZUR KONSTRUKTION EINER DIGITALEN, DREI-DIMENSIONALEN BESCHREIBUNG EINES PATIENTENINDIVIDUELLEN ZAHNERSATZES**

(71) Anmelder: Müller, Dirk, 65193 Wiesbaden (DE)
(72) Erfinder: Müller, Dirk, 65193 Wiesbaden (DE)
(74) Vertreter: WSL Patentanwälte Partnerschaft mbB

(57) **Zusammenfassung**

Die Erfindung betrifft ein computerimplementiertes Verfahren zur Konstruktion einer digitalen, dreidimensionalen Beschreibung eines patientenindividuellen Zahnersatzes. Hierzu wird ein vorab definiertes Schichtmodell mit mindestens zwei Schichten - Basisschicht, optionaler Übergangsschicht und Außenschicht - ausgewählt, das einen gewünschten visuellen Gesamteindruck liefert. Mittels einer Bildregistrierung der Innen- und Außenflächen des Modells auf patientenspezifische Zahngeometrien wird eine Transformation bestimmt, welche die Form anpasst und dabei die charakteristischen Schichtverhältnisse erhält. Die erzeugte Beschreibung dient zur Fertigung eines Zahnersatzes mit naturgetreuem visuellem Eindruck. Das Verfahren ermöglicht eine hochästhetische, digital unterstützte Zahnersatzgestaltung mit optimierter Fertigungskompatibilität, insbesondere für additive Herstellungsverfahren.

## Beschreibung

Die vorliegende Erfindung betrifft ein computerimplementiertes Verfahren zur Konstruktion einer digitalen, dreidimensionalen Beschreibung eines patientenindividuellen Zahnersatzes. Darüber hinaus betrifft die vorliegende Erfindung ein Verfahren zur Herstellung eines Zahnersatzes unter Verwendung einer mit dem Verfahren konstruierten Beschreibung.

Bei der herkömmlichen Fertigung eines Zahnersatzes wird zunächst eine präzise Abformung des präparierten Zahnstumpfs mittels Abformmaterial erstellt. Aus diesem Abdruck wird ein Gipsmodell gefertigt, das den exakten Stumpf und die umgebenden Nachbarzähne abbildet. Auf diesem Modell formt der Zahntechniker einen Wachsaufbau, der die spätere Anatomie des Zahnersatzes grob vorgibt. Anschließend wird das Wachsmodell in eine Feingusslegierung invertiert und das Wachs ausgebrannt, um Platz für das Metall oder die Keramikfüllung zu schaffen. Nach dem Guss bzw. der Presskeramikbearbeitung wird das Gerüst entnommen, gereinigt und bei Bedarf bemalt oder verblendet.

In der Anpassungsphase erfolgt am Patienten zunächst eine Probeanprobe des gefrästen oder gegossenen Gerüstes auf dem Stumpf. Kleinste Druckstellen werden durch selektives Abschleifen am Gerüst oder am Stumpf ausgeglichen, um eine exakte Randdichtigkeit und Passgenauigkeit sicherzustellen. Danach wird auf das Gerüst eine Verblendung aus Keramik oder Kunststoff aufgebracht und damit der Zahnersatz vollendet. Abschließend wird der Zahnersatz mittels Zahnzement oder Adhäsivverfahren fest auf den Stumpf zementiert, d.h. mit dem Kauapparat des Patienten verbunden.

Neben der einwandfreien Funktion des Zahnersatzes, d.h. der Möglichkeit, mit diesem Zahnersatz Nahrung zu zerteilen und zu mahlen, ist der optische Eindruck des Zahnersatzes wesentlich für die Zufriedenheit des Patienten mit dem Zahnersatz. Herkömmlicher Zahnersatz beruht typischerweise auf einem einzigen Material für die Verblendung, wobei dessen Farbe vom Zahnarzt oder Zahntechniker anhand eines Vergleichs von Mustern mit den natürlichen, im Mund des Patienten verbliebenen Nachbarzähnen bestimmt und festgelegt wird. Es hat sich aber gezeigt, dass Verblendungen aus nur einem einzigen Material nie den visuellen Gesamteindruck des ursprünglichen zu ersetzenden Zahns naturrealistisch annähern können. Dies ist der Tatsache geschuldet, dass natürliche Zähne einen vergleichsweise komplexen Aufbau aufweisen. Die einzelnen Schichten übernehmen nicht nur funktionell unterschiedliche Aufgaben, sondern tragen auch jede zum visuellen Gesamteindruck des vollständigen Zahns bei.

Daher sind aus dem Stand der Technik Verfahren zum Konstruieren und Herstellen von Zahnersatz bekannt, die auf einer Anordnung einer Mehrzahl von geschichteten Materialien beruhen. So beschreibt die Patentanmeldung EP 1 252 867 A1 ein CAD/CAM-gestütztes Verfahren zur automatisierten Berechnung und Herstellung einer Schichtstruktur der Verblendung eines Zahnersatzes. Um das natürliche Erscheinungsbild exakt nachzubilden, werden mehrere Schichten mit unterschiedlichen optischen Eigenschaften eingesetzt. Die optische Wirkung ergibt sich aus der Kombination der Schichten und dem Dickenverhältnis dieser Schichten. Die optische Wirkung wird mathematisch modelliert, um das gewünschte Farberscheinungsbild zu erreichen. Über eine angenommene lineare Additivität der Beiträge der einzelnen Schichten wird regional die optimale Dicke jeder einzelnen Schicht ermittelt.

Gegenüber dem Stand der Technik ist es eine Aufgabe der vorliegenden Erfindung, ein computerimplementiertes Verfahren zur Konstruktion einer digitalen dreidimensionalen Beschreibung eines Zahnersatzes für einen zu ersetzenden Zahn bereitzustellen, das es ermöglicht nach der Beschreibung einen Zahnersatz herzustellen, dessen visueller Gesamteindruck dem der natürlichen, individuellen Zähne des betroffenen Patienten näher kommt als vergleichbarer Zahnersatz aus dem Stand der Technik.

Zudem ist es eine Aufgabe der vorliegenden Erfindung, ein computerimplementiertes Verfahren bereitzustellen, welches in seiner Handhabung für den Benutzer, d. h. insbesondere für einen Zahntechniker, eine große Ähnlichkeit mit den eingeübten, konventionellen Verfahrensschritten zur Konstruktion und Herstellung eines Zahnersatzes aufweist.

Zumindest eine der zuvor genannten Aufgaben wird erfindungsgemäß durch ein computerimplementiertes Verfahren nach Anspruch 1 gelöst. Dazu weist das computerimplementierte Verfahren zur Konstruktion einer digitalen, dreidimensionalen Beschreibung eines patientenindividuellen Zahnersatzes für einen zu ersetzenden Zahn die Schritte auf:
A) Bereitstellen mindestens eines Schichtmodells,
   wobei jedes Schichtmodell eine Basisschicht und eine Außenschicht aufweist, wobei die Basisschicht einen ersten Parametersatz mit einem ersten Grundfarbton, einer ersten Opazität, einer Basisgrenzfläche und einer Modellinnenfläche aufweist, wobei die Außenschicht auf der Basisgrenzfläche oder einer Übergangsschichtgrenzfläche aufliegt,
   wobei die Außenschicht einen zweiten Parametersatz mit einem zweiten Grundfarbton, einer zweiten Opazität und einer Modellaußenfläche aufweist und
   wobei das Schichtmodell einen zumindest durch den ersten Parametersatz und den zweiten Parametersatz bestimmten Gesamteindruck der Modellaußenfläche aufweist;
B) Ableiten der Beschreibung des Zahnersatzes aus dem ausgewählten Schichtmodell mit den Schritten:
   i) Festlegen einer Zahnersatzinnenfläche des Zahnersatzes,
   ii) Festlegen einer Zahnersatzaußenfläche des Zahnersatzes,
   iii) Ausführen einer Bildregistrierung der Modellaußenfläche auf die Zahnersatzaußenfläche und der Modellinnenfläche auf die Zahnersatzinnenfläche, sodass eine Transformation bestimmt wird, welche die Modellaußenfläche mit einer minimierten Abweichung mit der Zahnersatzaußenfläche und die Modellinnenfläche mit einer minimierten Abweichung mit der Zahnersatzinnenfläche in Übereinstimmung bringt,
   iv) Anwenden der Transformation zumindest auf die Basisgrenzfläche, sodass eine Zahnersatzbasisgrenzfläche berechnet wird;
C) Speichern der Beschreibung des Zahnersatzes zur Verwendung bei der Herstellung des Zahnersatzes, wobei die Beschreibung zumindest eine Definition der Zahnersatzaußenfläche oder einer aus der Modellaußenfläche abgeleiteten Außenfläche, eine Definition der Zahnersatzinnenfläche oder einer aus der Modellinnenfläche abgeleiteten Innenfläche und eine Definition der Zahnersatzbasisgrenzfläche umfasst.

Es ist die grundlegende Idee der vorliegenden Erfindung, mindestens ein vorab definiertes Schichtmodell, vorzugsweise aber eine Mehrzahl von vorab definierten Schichtmodellen in einer Datenbank, mit jeweils mindestens zwei Schichten als Grundkonfiguration des Zahnersatzes bereitzustellen. Dabei erzeugt jedes dieser Schichtmodelle aufgrund seiner Schichtung einen eigenen visuellen Gesamteindruck. Fertigte man einen Rohling nach einem dieser Schichtmodelle so hätte dieser einen durch Farbe und Transparenz vorgegebenen visuellen Gesamteindruck aber eine nur grob an einen menschlichen Zahn angepasste Form.

Ein Schichtmodell im Sinne der vorliegenden Anmeldung ist ein Datensatz, welcher mittels mindestens zweier Schichten einen visuellen Gesamteindruck eines geometrisch stark vereinfachten, modellhaften Zahnersatzes definiert. Würde man einen Zahnersatz nach einem solchen Schichtmodell fertigen, so käme eine Art Rohling bzw. ein geometrisch stark vereinfachter Zahnersatz heraus, der aber abgesehen von seiner genauen Form im Hinblick auf die Farbe und Transparenz den Gesamteindruck eines herzustellenden Zahnersatzes wiedergibt. Dieser Gesamteindruck ist aufgrund der Schichtung des Modells sehr nah an demjenigen realer, zu ersetzender Zähne.

Der visuelle Gesamteindruck eines jeden Schichtmodells ist durch die quantifizierten Parameters-ätze der einzelnen Schichten sowie deren räumliche Anordnung und Dicke definiert. Der Visuelle Gesamteindruck ist damit eine objektiv messbare technische Eigenschaft. Die zunächst optionale Auswahl eines für einen spezifischen Patientenfall passenden Schichtmodells aus einer Mehrzahl von Schichtmodellen jedoch kann durch einen visuellen Abgleich durch einen Anwender des Verfahrens erfolgen - analog zum herkömmlichen Vergleich mit einer Farbmusterpalette. Alternativ kann diese Auswahl auch automatisiert, beispielsweise anhand von Bilddaten des zu ersetzenden Zahns oder der Nachbarzähne, erfolgen.

In einer Ausführungsform wird in Schritt A) eine Mehrzahl von Schichtmodellen in einer Datenbank bereitgestellt wird, wobei das Verfahren weiterhin den Schritt aufweist
D) Auswählen eines Schichtmodells (8) aus der Mehrzahl von Schichtmodellen in der Datenbank anhand eines für den Zahnersatz zu erzielenden visuellen Gesamteindrucks.

Das Schichtmodell oder die Mehrzahl von Schichtmodellen wird beispielsweise von einem Softwareanbieter oder dem Anbieter der entsprechenden Maschine zum Fertigen des konstruierten Zahnersatzes bereitgestellt. Ein Schichtmodell kann auf verschiedene Weisen generiert werden. Beispielsweise ist es möglich, ein Schichtmodell anhand eines beispielhaft extrahierten Zahnes, dessen Schichtaufbau bekannt ist, zu definieren. In einer Ausführungsform werden auch die Schichtmodelle softwaregestützt generiert.

In einer Ausführungsform werden die für ein Schichtmodell erforderlichen Informationen durch eine Vermessung, insbesondere durch eine spektrophotometrische Vermessung eines natürlichen Referenzzahns gewonnen, dessen Schichtaufbau zuvor - beispielsweise durch Schliffbildanalyse - ermittelt wurde.

Ein Schichtmodell kann beispielsweise durch einen 3D-Scan eines extrahierten Referenzzahns mit bekanntem Schichtaufbau und anschließende Schichtanalyse (z.B. mittels Schliffbildanalyse oder spektrophotometrischer Vermessung) gewonnen werden. Dabei werden die geometrischen Grenzen und die optischen Parameter (Grundfarbton, Opazität) jeder Schicht präzise erfasst und digitalisiert. Alternativ können Schichtmodelle softwaregestützt generiert werden, indem Materialparameter (z.B. C-Lab-Werte, Opazitätsgrade) und geometrische Layer-Profile (z.B. Dickenverläufe, Krümmungen) synthetisch definiert und zu einem digitalen Modell kombiniert werden, das einen spezifischen visuellen Gesamteindruck simuliert und validiert wird.

In einer Ausführungsform weist das Schichtmodell oder die Mehrzahl von Schichtmodellen mindestens drei Schichten auf. In einer Ausführungsform weist zumindest ein Schichtmodell mindestens eine Übergangsschicht aufweist,
wobei die mindestens eine Übergangsschicht auf der Basisgrenzfläche aufliegt,
wobei die mindestens eine Übergangsschicht einen dritten Parametersatz mit einem dritten Grundfarbton, einer dritten Opazität und einer Übergangsschichtgrenzfläche aufweist, wobei zumindest der dritte Grundfarbton von dem ersten Grundfarbton verschieden ist oder die dritte Opazität von der ersten Opazität verschieden ist und
wobei in Schritt iv) die Transformation auf die Übergangsschichtgrenzfläche jeder Übergangsschicht angewendet wird, so dass jeweils eine Zahnersatzübergangsschicht berechnet wird.

Dabei kann das Schichtmodell auch mehr als drei Schichten aufweisen. Insbesondere kann eine Mehrzahl von Übergangsschichten vorgesehen sein. Die Schichten jedes Schichtmodells sind einzigartig geformt, wobei die Schichten zueinander definierte räumliche Lagebeziehungen aufweisen. Beispielsweise kann die Schichtdicke der mindestens einen Übergangsschicht über das Zahnvolumen hinweg variieren. Es hat sich gezeigt, dass mindestens drei Schichten notwendig sind, um einen natürlichen Gesamteindruck des Zahnersatzes hervorzurufen.

In einer Ausführungsform der Erfindung unterscheiden sich mindestens zwei aus der Mehrzahl von Schichtmodellen auch durch die Zahnart, die ein nach dem Schichtmodell nachgearbeiteter Zahnersatz ersetzen soll. Schichtmodelle für unterschiedliche Arten von Zähnen unterscheiden sich nicht nur durch die relativen Dicken und Formen der Schichten voneinander, sondern auch durch die Grundform des jeweiligen Schichtmodells. Beispielsweise gibt es in einer Ausführungsform mindestens ein Schichtmodell für einen jugendlichen Zahn, mindestens ein Schichtmodell für einen älteren Zahn und mindestens ein Schichtmodell für einen Zahn mit oberflächlichen Verfärbungen.

In einer Ausführungsform der Erfindung unterscheiden sich mindestens zwei aus der Mehrzahl von Schichtmodellen durch die spezifische Zahnzustände (z.B. jugendlicher Zahn, älterer Zahn, Zahn mit oberflächlichen Verfärbungen) des Zahns, den ein nach dem Schichtmodell nachgearbeiteter Zahnersatz ersetzen soll.

In einer Ausführungsform der Erfindung belegt die Übergangsschicht die Basisgrenzfläche vollständig. In einer anderen Ausführungsform bildet die Übergangsschicht eine oder mehre Inseln auf der Basisgrenzfläche, beispielsweise um Kalkeinschlüsse in einem Zahn nachzubilden.

In einer Ausführungsform der Erfindung sind die Grundfarbtöne der Schichten quantifiziert, z.B. in C-Lab-Koordinaten (L*, a*, b*). In einer weiteren Ausführungsform der Erfindung sind die Opazitäten der einzelnen Schichten quantifiziert, beispielsweise in Prozent.

Im Sprachgebrauch der vorliegenden Anmeldung bezeichnen die Grenzflächen der Schichten solche Flächen, auf denen eine oder mehrere andere Schichten aufliegen.

Die Modellinnenfläche ist diejenige Fläche, mit der sich das Schichtmodell in Abhängigkeit von der Ausgestaltung des Zahnersatze auf einem Gerüst oder aber direkt auf einem Abutment oder einem Zahnstumpf abstützen würde, wenn man einen nach dem Schichtmodell gefertigten Rohling in ein Gebiss einsetzen würde. Stützt sich die Modellinnenfläche und somit auch die Zahnersatzinnenfläche auf einem Gerüst ab, so bildet die Zahnersatzinnenfläche die Innenfläche der Verblendung. Stützt sich die Modellinnenfläche und somit auch die Zahnersatzinnenfläche auf einem Abutment oder einem Zahnstumpf ab, so bildet sie die Innenfläche des gesamten Zahnersatzes.

Die Modellaußenfläche hingegen ist diejenige Fläche, die dann, wenn man einen Rohling nach dem Schichtmodell fertigt, die im eingesetzten Zustand sichtbare Außenfläche des Rohlings bildet.

Der Nutzer des computerimplementierten Verfahrens, d. h. insbesondere einer Software, beispielsweise ein Zahntechniker, wählt in einer Ausführungsform in Schritt D) dasjenige Schichtmodell aus der Mehrzahl von Schichtmodellen aus der Datenbank aus, von dem er davon ausgeht, dass es im Hinblick auf seinen visuellen Gesamteindruck dem visuellen Gesamteindruck des zu ersetzenden Zahns am nächsten kommt. Dieser Schritt ist dem Vergleich einer Musterpalette mit den Zähnen des Patienten, so wie er herkömmlich erfolgt, sehr ähnlich.

In einer Ausführungsform jedoch erfolgt das Auswählen des Schichtmodells aus der Mehrzahl von Schichtmodellen automatisiert, wobei ein Bild des noch nicht extrahierten zu ersetzenden Zahnes oder ein Bild der Nachbarzähne für das automatisierte Auswählen herangezogen wird.

Bei einem solchen Schichtmodell weisen die Modellinnenfläche und die Modellaußenfläche jeweils eine Grundform auf, so wie sie bei einem Zahnstumpf oder Abutment (Modellinnenfläche), einem Zahn (Modellaußenfläche) typischerweise vorkommen. Diese Grundformen sind aber in keiner Weise individuell auf den jeweiligen Patienten angepasst.

Man geht nun davon aus, dass der visuelle Gesamteindruck, so wie ihn das jeweilige Schichtmodell beschreibt und den ein nach dem Schichtmodell gefertigter Rohling hätte, für die Fertigung des Zahnersatzes beizubehalten ist. Der herzustellende Zahnersatz unterscheidet sich im Hinblick auf seine Form aber durch eine Abweichung der herzustellenden Zahnersatzaußenfläche des Zahnersatzes von der Modellaußenfläche und durch eine Abweichung der Zahnersatzinnenfläche des Zahnersatzes von der Modellinnenfläche.

Daher werden beim Ableiten der Beschreibung des Zahnersatzes aus dem Schichtmodell zunächst die Zahnersatzinnenfläche des Zahnersatzes und die Zahnersatzaußenfläche des Zahnersatzes festgelegt.

Für das Festlegen der Zahnersatzaußenfläche wird in einer Ausführungsform der Erfindung ein Bild des zu ersetzenden Zahnes verwendet. In einer alternativen Ausführungsform wird ein Bild der den zu ersetzenden Zahn umgebenden Zähne, d.h. der benachbarten Zähne und/oder des Antagonisten verwendet.

Das Festlegen der Zahnersatzinnenfläche erfolgt in einer Ausführungsform anhand eines dreidimensionalen Bildes eines Abdrucks eines präparierten Zahnstumpfs oder eines Abutments.

In einer alternativen Ausführungsform erfolgt das Festlegen der Zahnersatzinnenfläche, so wie es an späterer Stelle in dieser Anmeldung dargelegt wird, anhand eines vom Benutzer bereitgestellten Gerüsts bzw. dessen digitaler Beschreibung. In einer Ausführungsform mit einem Gerüst definiert die erfindungsgemäße Beschreibung des Zahnersatzes den typischerweise als Verblendung bezeichneten Abschnitt eines Zahnersatzes.

Die vorliegende Erfindung geht davon aus, dass der Gesamteindruck des Schichtmodells bzw. eines auf Grundlage des Schichtmodells gefertigten Rohlings sich dann erhalten lässt, wenn bei einer Anpassung des Modells an die Zahnersatzaußenfläche und die Zahnersatzinnenfläche die relativen Schichtdicken und Formen der einzelnen Schichten des Schichtmodells erhalten bleiben. Daher umfasst das Ableiten der Beschreibung des Zahnersatzes aus dem ausgewählten Schichtmodell ein Ausführen einer Bildregistrierung sowohl der Modellaußenfläche auf die Zahnersatzaußenfläche als auch der Modellinnenfläche auf die Zahnersatzinnenfläche. Die Bildregistrierung bestimmt eine Transformation, welche die Modellaußenfläche mit einer minimierten Abweichung mit der Zahnersatzaußenfläche und die Modellinnenfläche mit einer minimierten Abweichung mit der Zahnersatzinnenfläche in Übereinstimmung bringt. In einer Ausführungsform erfolgt die Bildregistrierung der Modellaußenfläche auf die Zahnersatzaußenfläche und der Modellinnenfläche auf die Zahnersatzinnenfläche gleichzeitig.

Eine Bildregistrierung wird auf dem Gebiet der Bildverarbeitung auch als Morphing, d. h. die Anpassung eines Objektbildes an ein Referenzbild, bezeichnet. Bei der erfindungsgemäßen Bildregistrierung bilden die Zahnersatzaußenfläche und die Zahnersatzinnenfläche das Referenzbild, während die Modellaußenfläche und die Modellinnenfläche das Objektbild bilden. Eine Bildregistrierung dient dazu, zwei Bilder des gleichen Objekts, nämlich das Objektbild und das Referenzbild, bestmöglich in Übereinstimmung miteinander zu bringen.

In einer Ausführungsform der Erfindung wird genau eine Transformation bestimmt. In einer Ausführungsform der Erfindung erfolgt das Bestimmen der Transformation in Schritt iii) derart, dass die Transformation die topologische Struktur der Zahnersatzaußenfläche und der Zahnersatzinnenfläche erhält.

In einer Ausführungsform der Erfindung erfolgt das Bestimmen der Transformation in Schritt iii) derart, dass die relativen Dicken der Basisschicht, der Außenschicht und optional der mindestens einen Übergangsschicht in der Zahnersatzbasisschicht und der Zahnersatzaußenschicht sowie optional der mindestens einen Zahnersatzübergangsschicht erhält.

In einer weiteren Ausführungsform der Erfindung erfolgt das Bestimmen der Transformation in Schritt iii) derart, dass die Transformation die Lagebeziehungen und Orientierungsbeziehungen der Basisgrenzfläche und der mindestens einen Übergangsschichtgrenzfläche innerhalb eines vorbestimmten relativen Toleranzbereichs in der Zahnersatzbasisgrenzfläche und der mindestens einen Zahnersatzübergangsschichtgrenzfläche erhält.

In einer Ausführungsform ist der Toleranzbereich derart gewählt, dass die charakteristischen visuellen Merkmale der Schichten erhalten bleiben.

In einer weiteren Ausführungsform ist die Transformation kontinuierlich. In einer Ausführungsform der Erfindung ist die Bildregistrierung nicht-rigide. Ein nicht-rigides Registrierungsverfahren ist ein Verfahren, bei dem die Objektfläche und die Referenzfläche ineinander überführt werden, wobei lokale Verformungen erlaubt sind. Ein solches nicht-rigides Verfahren ist vorteilhaft, da nicht davon auszugehen ist, dass natürliche Flächen als Referenzfläche nur durch Verschiebung, Drehung oder Skalierung der einzelnen Punkte auf der Objektfläche erreicht werden können.

In einer Ausführungsform der Erfindung erfolgt das Bestimmen der Transformation in Schritt iii) mit einem, vorzugsweise nicht-rigiden Coherent-Point-Drift-Algorithmus.

In einer Ausführungsform der Erfindung berücksichtigt der Coherent-Point-Drift-Algorithmus den geodätischen Abstand zwischen einer Mehrzahl von Punkten auf der Zahnersatzaußenfläche.

In einer Ausführungsform der Erfindung wird als Abweichung zwischen dem Objektbild und dem Referenzbild eine mittlere Flächenabweichung zwischen der Modellaußenfläche und der Zahnersatzaußenfläche sowie der Modellinnenfläche und der Zahnersatzinnenfläche betrachtet. In einer Ausführungsform der Erfindung beträgt die mittlere Flächenabweichung 5 % oder weniger, bevorzugt 3 % oder weniger.

In einer Ausführungsform der Erfindung wird während der morphologischen Anpassung sichergestellt, dass die Schichtdicken entlang der lokalen Normalenrichtungen zwischen zwei Flächen einer Schicht, z.B. die Dicke der Basisschicht zwischen der Modellinnenfläche und der Basisgrenzfläche, nur innerhalb eines vorbestimmten relativen Toleranzbereichs verändert werden. Dieser relative Unterschied im Abstand - bezogen auf den ursprünglichen Abstand vor der Anpassung - beträgt vorzugsweise höchstens Δd = 5 %, besonders bevorzugt höchstens Δd = 3 %. Δd bezeichnet dabei die lokale, relative Veränderung des Schichtabstands an einem Punkt, gemessen entlang der zugehörigen lokalen Normalenrichtung, und stellt keine gemittelte Flächenabweichung dar.

Die auf diese Weise bestimmte Transformation erhält die räumlichen Lagebeziehungen und Orientierungsbeziehungen der Grenzflächen der Schichten eines Schichtmodells zueinander, wenn die Transformation auf die Grenzflächen des Schichtmodells anwendet. Die Transformierte des Schichtmodells hat daher im Wesentlichen den gleichen visuellen Gesamteindruck wie das Schichtmodell.

Daher wird erfindungsgemäß gewonnene Transformation zumindest auf die Basisgrenzfläche und optional auf die mindestens eine Übergangsschichtgrenzfläche, vorzugsweise auf alle Übergangsschichtgrenzflächen, wenn es eine Mehrzahl von Übergangsschichten gibt, angewendet. Wenn es eine Mehrzahl von Übergangsschichten gibt, so wird die Transformation auf jeder aus der Mehrzahl von Übergangsschichtgrenzflächen angewendet. Durch die Anwendung der Transformation wird aus der Basisgrenzfläche eine Zahnersatzbasisgrenzfläche und optional aus jeder Übergangsschichtgrenzfläche eine Zahnersatzübergangsschichtgrenzfläche berechnet.

Es ist möglich, nur die Grenzflächen durch Anwenden der Transformation in entsprechende Zahnersatzgrenzflächen zu überführen, und damit bereits ein qualitativ hochwertiges Ergebnis für den Zahnersatz zu erzielen.

Dennoch wird in einer Ausführungsform der Erfindung die Transformation auch auf die Modellaußenfläche angewandt, so dass eine abgeleitete Außenfläche erhalten wird, und auf die Modellinnenfläche angewandt, so dass eine abgeleitete Innenfläche erhalten wird. Ein solches Vorgehen sorgt dafür, dass die relativen Schichtdicken und räumlichen Relationen des ursprünglich gewählten Schichtenmodells in der Beschreibung des Zahnersatzes noch originalgetreuer erhalten bleiben.

Es versteht sich, dass in einer solchen Ausführungsform die Beschreibung des Zahnersatzes zur Verwendung bei dessen Herstellung die abgeleitete Außenfläche und die abgeleitete Innenfläche umfasst.

In einer Ausführungsform der Erfindung werden die Schichten des Zahnersatzes auf ein Gerüst des Zahnersatzes aufgebracht. Daher weist das Verfahren in einer Ausführungsform zusätzlich die Schritte auf:
E) Bereitstellen eines Gerüstmodells mit einer Gerüstinnenfläche und einer Gerüstaußenfläche,
   wobei die Gerüstaußenfläche von einer Zahnart des zu ersetzenden Zahnes abhängt;
F) Erfassen einer Geometrie eines individuellen Zahnstumpfs eines Patienten mit einem bildgebenden Verfahren oder
   Definieren einer Außenkontur eines Abutments, welches den Zahnersatz mit einem Kiefer des Patienten verbindet; und
G) Anpassen der Innenfläche des Gerüstmodells, sodass die Innenfläche eine Aufnahme für den Zahnstumpf oder das Abutment ausbildet;
   wobei die in Schritt i) festgelegte Zahnersatzinnenfläche oder die aus der Modellinnenfläche abgeleitete Innenfläche die Gerüstaußenfläche ist.

In einer Ausführungsform erfolgt das Erfassen der Geometrie des Zahnstumpfs mit einem intraoralen 3-D-Scanner.

In einer Ausführungsform der Erfindung kommt der Zahnersatz ohne ein Gerüst aus. In einer solchen Ausführungsform wird die Modellinnenfläche der Basisschicht unmittelbar an das Bild eines Zahnstumpfs oder eines Abutments angepasst.

In einer Ausführungsform der Erfindung umfasst jedes aus der Mehrzahl von Schichtmodellen Volumendaten für die Basisschicht, optional für die mindestens eine Übergangsschicht und für die Außenschicht und/oder die Beschreibung des Zahnersatzes umfasst Volumendaten für die von der Zahnersatzbasisgrenzfläche begrenzte Zahnersatzbasisschicht und optional für die mindestens eine von der Zahnersatzübergangsschichtgrenzfläche begrenzte Zahnersatzübergangsschicht.

In einer Ausführungsform der Erfindung erfolgt das Festlegen der Zahnersatzaußenfläche in Schritt i) unter Berücksichtigung zumindest eines 3D-Scans des zu ersetzenden Zahnes oder eines 3D-Scans eines dem zu ersetzenden Zahn benachbarten Zahns.

In einer weiteren Ausführungsform der Erfindung erfolgt das Festlegen der Zahnersatzaußenfläche in Schritt i) durch Auswählen einer geeigneten Zahnform aus einer Datenbank und einer anschließenden Anpassung an die individuelle Zahngeometrie des zu ersetzenden Zahns.

In einer Ausführungsform der Erfindung weist jedes der Schichtmodelle eine Mehrzahl von Übergangsschichten auf, wobei die Beschreibung des Zahnersatzes eine Definition einer Mehrzahl von Zahnersatzübergangsschichtgrenzflächen umfasst.

In einer Ausführungsform der Erfindung ist die Außenschicht eine Effektschicht. Eine Effektschicht bezeichnet in der Zahntechnik eine Schicht, die aufgrund ihrer optischen Eigenschaften einen besonderen Effekt beim Betrachter des Zahnersatzes generiert. In einer Ausführungsform ist die Effektschicht eine pigmentierte oder eine transluzente Schicht. Ein Beispiel für eine Effektschicht ist eine Schneidekanten-Effektmasse. Dabei ist eine Effektschicht in einer Ausführungsform eine dünne Schicht. In einer Ausführungsform ist die Effektschicht dünner als zumindest die Basisschicht oder die oder die Mehrzahl von Übergangsschichten.

In einer Ausführungsform der Erfindung ist der dritte Grundfarbton von dem zweiten Grundfarbton verschieden.

In einer Ausführungsform ist die dritte Opazität kleiner als die zweite Opazität.

Insoweit die oben beschriebenen Ausführungsformen des Verfahrens zumindest teilweise realisiert werden können, wobei eine softwaregesteuerte Datenverarbeitungsvorrichtung verwendet wird, ist es offensichtlich, dass ein Computerprogramm, das solch eine Softwaresteuerung bereitstellt, und eine Datenverarbeitungsvorrichtung, die Mittel zur Ausführung des Verfahrens sowie eine Ausgabeeinheit zum Bereitstellen der Beschreibung des Zahnersatzes umfasst, als Aspekte der Erfindung in Betracht zu ziehen sind.

Zumindest eine der zuvor genannten Aufgaben wird auch durch ein Verfahren zur Herstellung eines Zahnersatzes gemäß dem Anspruch 14 gelöst. Dazu umfasst das Verfahren zur Herstellung des Zahnersatzes die Schritte:
Bereitstellen der Beschreibung des Zahnersatzes mit einem computerimplementierten Verfahren nach einer der zuvor beschriebenen Ausführungsformen, wobei das Fertigen zumindest der mindestens einen Übergangsschicht oder der Außenschicht additiv erfolgt.

In einer Ausführungsform der Erfindung erfolgt das Fertigen der Basisschicht additiv oder subtraktiv. In einer Ausführungsform der Erfindung kann nach dem additiven Fertigen optional eine subtraktive Bearbeitung zur Korrektur einer Schicht folgen.

Weitere Vorteile, Merkmale und Anwendungsmöglichkeiten der vorliegenden Erfindung werden anhand der folgenden Beschreibung einer Ausführungsform und der dazugehörigen Figuren deutlich. In den Figuren sind gleiche Elemente mit identischen Bezugszeichen bezeichnet.
- Figur 1: ist eine schematische Seitenansicht eines Zahnstumpfs mit einem aufgesetzten Gerüst.
- Figur 2: ist eine um 90 Grad gegenüber der Figur 1 gedrehte schematische Seitenansicht des Zahnstumpfs mit dem aufgesetzten Gerüst.
- Figur 3: ist eine schematische, teilweise transparente Seitenansicht des Zahnstumpfs und des Gerüsts aus Figuren 1 und 2 mit einer Zahnersatzaußenfläche.
- Figur 4: ist eine um 90 Grad gedrehte schematische Seitenansicht der Anordnung aus Figur 3.
- Figur 5: ist eine schematische Querschnittsansicht der Anordnung aus den Figuren 3 und 4.
- Figur 6: ist eine schematische Schnittansicht eines erfindungsgemäßen Schichtmodells.
- Figur 7: ist eine gegenüber Figur 6 um 90 Grad gedrehte schematische Seitenansicht des Schichtmodells aus Figur 6.
- Figur 8: ist eine schematische Querschnittsansicht des Schichtmodells aus den Figuren 6 und 7.
- Figur 9: ist eine schematische Darstellung der erfindungsgemäßen Bildregistrierung.
- Figur 10: ist eine gegenüber Figur 9 um 90 Grad gedrehte Darstellung der Bildregistrierung aus Figur 9.
- Figur 11: ist eine schematische Querschnittsansicht der Bildregistrierung aus den Figuren 9 und 10.
- Figur 12: ist eine schematische Schnittansicht der Beschreibung des Zahnersatzes.
- Figur 13: ist eine um 90 Grad gedrehte Seitenansicht der Beschreibung des Zahnersatzes aus Figur 12.
- Figur 14: ist eine schematische Querschnittsansicht der Beschreibung des Zahnersatzes aus Figuren 12 und 13.

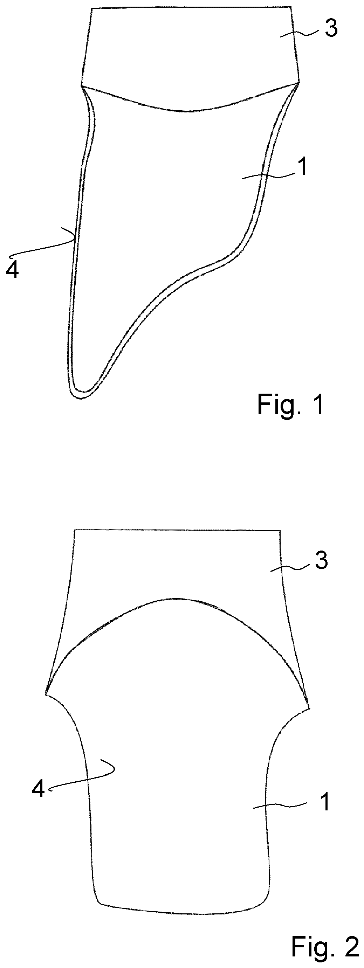

Die Figuren 1 und 2 zeigen ein Modell eines Gerüsts 1 für einen Zahnersatz, wobei das Gerüst 1 bereits auf einen Zahnstumpf 3 aufgesetzt dargestellt ist. Ein solches Gerüst 1 dient als Bindeglied zwischen dem Zahnstumpf 3 und einer Verblendung des Zahnersatzes. Das nach dem gezeigten Modell gefertigte Gerüst könnte auch als Träger für eine herkömmliche Verblendung eingesetzt werden.

Um die Beschreibung des Zahnersatzes mit dem erfindungsgemäßen Verfahren zu konstruieren, muss der Benutzer, im vorliegenden Beispiel ein Zahntechniker, zunächst ein Gerüstmodell, d. h. eine digitale Beschreibung des Gerüsts 1 bereitstellen. Das beschriebene Gerüst 1 weist eine Gerüstinnenfläche auf, welche komplementär zur Außenfläche eines individuellen Zahnstumpfs des zu ersetzenden Zahns ist. Dazu wird beispielsweise die Geometrie des realen Zahnstumpfs in der Mundhöhle des Patienten mit einem bildgebenden Verfahren erfasst und die Gerüstinnenfläche komplementär zu den präparierten Flächen des Zahnstumpfs 3 ausgebildet.

Die Gerüstaußenfläche 4 ist so bemessen, dass sie eine Grundform des zu ersetzenden Zahns widerspiegelt, aber dennoch genügend Platz lässt, um die Verblendung auf das Gerüst 1 aufzubringen. Mit anderen Worten ausgedrückt, ist das von der Gerüstaußenfläche 4 begrenzte Volumen kleiner als das von der Außenfläche des zu ersetzenden Zahns begrenzte Volumen.

In den Figuren 1 und 2 ist erkennbar, dass die Zahnart des zu ersetzenden Zahns die eines Schneidezahns ist. Die Form der Außenfläche 4 wird vom Zahntechniker anhand von Erfahrung und einem Bild der Nachbarzähne des zu ersetzenden Zahns sowie gegebenenfalls eines Bildes des zu ersetzenden Zahns ausgestaltet.

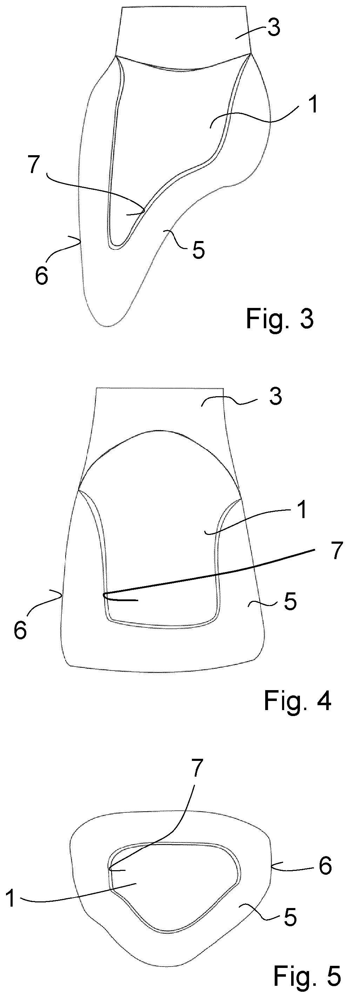

Die Figuren 3 bis 5 zeigen den Zahnersatz und den Stumpf 3 mit einer auf das Gerüst 1 aufgesetzten Verblendung 5. Dabei ist die Verblendung 5 nur schematisch dargestellt, in dem Sinne, dass nur die Zahnersatzaußenfläche 6 eingezeichnet ist. Die Zahnersatzaußenfläche 6 wird vom Zahntechniker in der das erfindungsgemäße Verfahren implementierenden Software festgelegt. Dabei kann das Festlegen der Zahnersatzaußenfläche 6 manuell erfolgen, so wie dies bei herkömmlichem Zahnersatz noch häufig der Fall ist. Mit seiner Erfahrung und Informationen aus Abdrücken und/oder Bildern über den zu ersetzenden Zahn sowie seine Nachbarzähne und Antagonisten kann der Zahntechniker die Zahnersatzaußenfläche 6 naturrealistisch gestalten und festlegen.

In einer Variante kann eine solche Festlegung der Zahnersatzaußenfläche 6 jedoch auch vollständig automatisiert durch Datenverarbeitung erfolgen. Am Ende der Festlegung der Zahnersatzaußenfläche 6 ist diese durch einen Datensatz beschrieben.

Wie aus den Figuren 3 bis 5 leicht ersichtlich ist, bildet die Gerüstaußenfläche 4 die Zahnersatzinnenfläche 7 der Verblendung 5.

Dem erfindungsgemäßen Verfahren folgend muss der Zahntechniker in einem nächsten Schritt ein Schichtmodell 8 auswählen.

Jedes dieser Schichtmodelle 8 beschreibt eine Mehrzahl von Schichten, welche zusammen die Verblendung 5 des herzustellenden Zahnersatzes bilden. Bei dem Schichtmodell handelt es sich um eine mathematische Beschreibung eines "Rohlings". Würde man die Verblendung 5 nach diesem Schichtmodell 8 herstellen, so erhielte man einen Formkörper mit einer Modellaußenfläche 9, die der Zahnersatzaußenfläche 6 ähnlich ist, diese aber nur grob verallgemeinert nachempfindet. Eine Abweichung zwischen der Zahnersatzaußenfläche 6 und der Modellaußenfläche 9 ist vergleichsweise groß. Zwar ist die Modellaußenfläche 9 der Zahnart des zu ersetzenden Zahns nachempfunden, sie hat allerdings keinerlei Bezug zu der individuellen Form der Außenfläche des zu ersetzenden Zahns bzw. der dieser nachempfundenen Zahnersatzaußenfläche 6, welche der Zahntechniker zuvor für den herzustellenden Zahnersatz festgelegt hat.

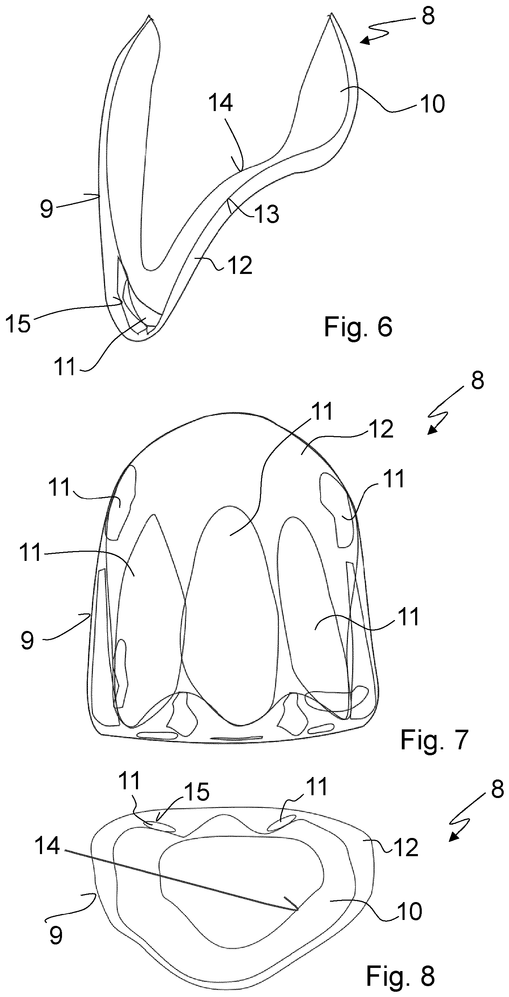

Das anhand der Figuren 6 bis 8 beschriebene Schichtmodell 8 umfasst drei Schichten, nämlich eine Basisschicht 10, eine Übergangsschicht 11 und eine Außenschicht 12. Jede der drei Schichten wird jeweils von einem Parametersatz beschrieben. Die Basisschicht weist einen ersten Parametersatz mit einem ersten Grundfarbton, einer ersten Opazität, einer Basisgrenzfläche 13 und einer Modellinnenfläche 14 auf. Die Übergangsschicht weist einen dritten Parametersatz mit einem dritten Grundfarbton, einer dritten Opazität und einer Übergangsschichtgrenzfläche 15 auf. Die Außenschicht weist einen zweiten Parametersatz mit einem zweiten Grundfarbton, einer zweiten Opazität und der Modellaußenfläche 9 auf. In dem beschriebenen Beispiel sind der erste, der zweite und der dritte Grundfarbton voneinander verschieden. Ferner sind die erste, die zweite und die dritte Opazität voneinander verschieden. Die Übergangsschicht 11 liegt auf der Basisgrenzfläche 13 auf. Die Außenschicht 9 liegt auf der Übergangsschichtgrenzfläche 15 der Übergangsschicht 11 auf. Da die Übergangsschicht 11 in dem gezeigten Beispiel die Basisgrenzfläche nicht vollständig bedeckt, sondern inselartig ausgebildet ist, liegt die Außenschicht 12 abschnittsweise auch auf der Basisgrenzfläche 13 der Basisschicht 10 auf.

Der erste, der zweite und der dritte Parametersatz zusammen bestimmen den visuellen Gesamteindruck der Modellaußenfläche 9. Der Zahntechniker wählt nun dasjenige Schichtmodell 8 aus der Mehrzahl von Schichtmodellen aus einer Datenbank aus, das den gewünschten, für den Zahnersatz zu erzielenden visuellen Gesamteindruck aufweist.

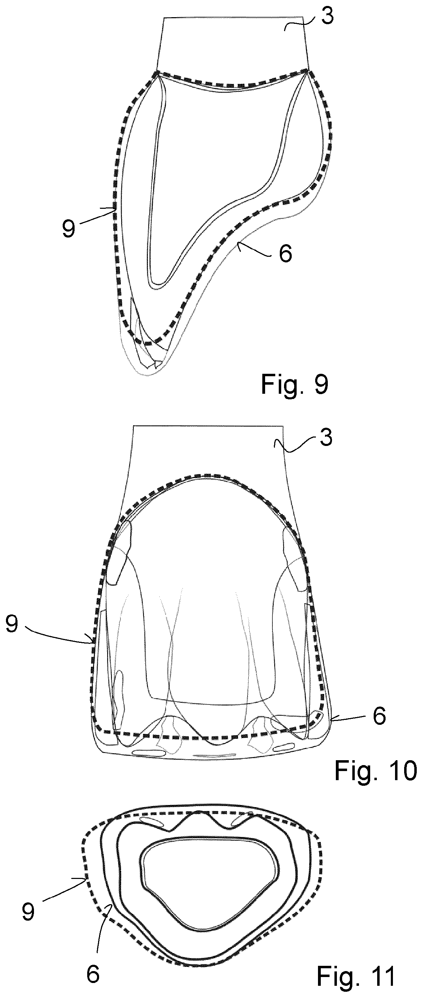

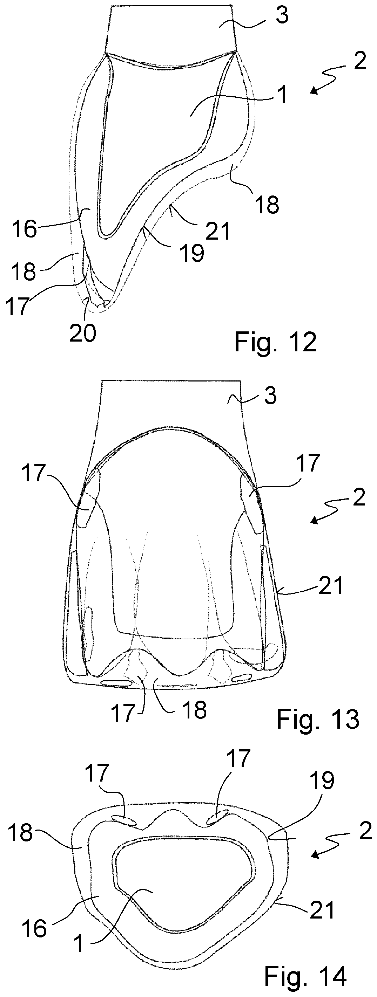

Diesen gewünschten Gesamteindruck gilt es nun auf den herzustellenden Gesamteindruck bzw. dessen digitale Beschreibung zu übertragen. Die Figuren 9 - 11 erläutern schematisch, wie das ausgewählte Schichtmodel an die individuelle Zahnform des Patienten, welche durch die Zahnersatzaußenfläche 6 verkörpert wird, unter Erhaltung des visuellen Gesamteindrucks des Schichtmodells 8 angepasst wird. Die Figuren 12 - 14 zeigen die dabei erzeugte, fertige digitale Beschreibung des Zahnersatzes 2.

Zunächst wird eine gleichzeitige Bildregistrierung der Modellaußenfläche 9 auf die Zahnersatzaußenfläche 6 und der Modellinnenfläche 14 auf die Zahnersatzinnenfläche 7 ausgeführt. Aus der Bildregistrierung ergibt sich eine einzige kontinuierliche, nicht-rigide Transformation, welche die Modellaußenfläche 9 mit einer minimierten Abweichung mit der Zahnersatzaußenfläche 6 und die Modellinnenfläche 14 mit einer minimierten Abweichung mit der Zahnersatzinnenfläche 7 in Übereinstimmung bringt.

In der exemplarisch dargestellten Variante folgt die Transformation einem nicht-rigiden Coherent-Point-Drift-Algorithmus, welcher den geodätischen Abstand zwischen einer Mehrzahl von Punkten auf der Zahnersatzaußenfläche berücksichtigt.

Die so bestimmte Transformation erhält, wenn man sie auf das Schichtmodell 8 anwendet die topologische Struktur der Zahnersatzaußenfläche 6 und der Zahnersatzinnenfläche 7 sowie die relativen Dicken der Basisschicht 10, der Übergangsschicht 11 und der Außenschicht 12 in der Zahnersatzbasisschicht 16, der Zahnersatzübergangsschicht 17 und der Zahnersatzaußenschicht 18. Zudem erhält die Transformation die Lagebeziehungen und Orientierungsbeziehungen der Basisgrenzfläche 13 und der Übergangsschichtgrenzfläche 15 innerhalb eines vorbestimmten relativen Toleranzbereichs von weniger als 3 % in der Zahnersatzbasisgrenzfläche 19 und der Zahnersatzübergangsschichtgrenzfläche 20.

In der diskutierten Variante wird die Transformation auch auf die Modellaußenfläche 9 angewandt, so dass eine abgeleitete Außenfläche 21 erhalten wird, und auf die Modellinnenfläche angewandt, so dass eine abgeleitete Innenfläche erhalten wird.

Die Beschreibung 2 des Zahnersatzes umfasst neben einer Definition der Zahnersatzbasisgrenzfläche19 , einer Definition der Zahnersatzübergangsschichtgrenzfläche 20 auch eine Definition der abgeleiteten Außenfläche 21 und der abgeleiteten Innenfläche. Mit dieser Beschreibung lässt sich ein für den Patienten individualisierter Zahnersatz fertigen, der den gleichen visuellen Gesamteindruck im Hinblick auf Farbe und Opazität aufweist, obwohl sich seine abgeleitete Außenfläche 21 erheblich von der Modellaußenfläche 9 unterscheidet.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschrieben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten Ausführungsformen beschränkt.

Abwandlungen der offenbarten Ausführungsformen sind für den Fachmann aus den Zeichnungen, der Beschreibung und den beigefügten Ansprüchen offensichtlich. In den Ansprüchen schließt das Wort "aufweisen" nicht andere Elemente oder Schritte aus, und der unbestimmte Artikel "eine" oder "ein" schließt eine Mehrzahl nicht aus. Die bloße Tatsache, dass bestimmte Merkmale in unterschiedlichen Ansprüchen beansprucht sind, schließt ihre Kombination nicht aus. Bezugszeichen in den Ansprüchen sind nicht als Beschränkung des Schutzbereichs gedacht.

### Bezugszeichenliste

- 1: Gerüst
- 2: Beschreibung des Zahnersatzes
- 3: Zahnstumpf
- 4: Gerüstaußenfläche
- 5: Verblendung
- 6: Zahnersatzaußenfläche
- 7: Zahnersatzinnenfläche
- 8: Schichtmodell
- 9: Modellaußenfläche
- 10: Basisschicht
- 11: Übergangsschicht
- 12: Außenschicht
- 13: Basisgrenzfläche
- 14: Modellinnenfläche
- 15: Übergangsschichtgrenzfläche
- 16: Zahnersatzbasisschicht
- 17: Zahnersatzübergangsschicht
- 18: Zahnersatzaußenschicht
- 19: Zahnersatzbasisgrenzfläche
- 20: Zahnersatzübergangsschichtgrenzfläche
- 21: abgeleitete Außenfläche

## Patentansprüche

1. Computerimplementiertes Verfahren zur Konstruktion einer digitalen, dreidimensionalen Beschreibung (2) eines patientenindividuellen Zahnersatzes für einen zu ersetzenden Zahn mit den Schritten
A) Bereitstellen mindestens eines Schichtmodells (8),
wobei jedes Schichtmodell (8) eine Basisschicht und eine Außenschicht (12) aufweist,
wobei die Basisschicht (10) einen ersten Parametersatz mit einem ersten Grundfarbton, einer ersten Opazität, einer Basisgrenzfläche (13) und einer Modellinnenfläche (14) aufweist,
wobei die Außenschicht (12) auf der Basisgrenzfläche oder einer Übergangsschichtgrenzfläche (15) einer Übergangsschicht (11) aufliegt,
wobei die Außenschicht (12) einen zweiten Parametersatz mit einem zweiten Grundfarbton, einer zweiten Opazität und einer Modellaußenfläche (9) aufweist und
wobei das Schichtmodell (8) einen zumindest durch den ersten Parametersatz und den zweiten Parametersatz bestimmten Gesamteindruck der Modellaußenfläche (9) aufweist;
B) Ableiten der Beschreibung (2) des Zahnersatzes aus dem ausgewählten Schichtmodell (8) mit den Schritten:
i) Festlegen einer Zahnersatzinnenfläche (7) des Zahnersatzes,
ii) Festlegen einer Zahnersatzaußenfläche (6) des Zahnersatzes,
iii) Ausführen einer Bildregistrierung der Modellaußenfläche (9) auf die Zahnersatzaußenfläche (6) und der Modellinnenfläche (14) auf die Zahnersatzinnenfläche (7), so dass eine Transformation bestimmt wird, welche die Modellaußenfläche (9) mit einer minimierten Abweichung mit der Zahnersatzaußenfläche (6) und die Modellinnenfläche (14) mit einer minimierten Abweichung mit der Zahnersatzinnenfläche (7) in Übereinstimmung bringt,
iv) Anwenden der Transformation zumindest auf die Basisgrenzfläche (13), so dass eine Zahnersatzbasisgrenzfläche (19) berechnet wird;
C) Speichern der Beschreibung (2) des Zahnersatzes zur Verwendung bei der Herstellung des Zahnersatzes, wobei die Beschreibung (2) zumindest eine Definition der Zahnersatzaußenfläche (6) oder einer aus der Modellaußenfläche (6) abgeleiteten Außenfläche, der Zahnersatzinnenfläche (7) oder einer aus der Modellinnenfläche (7) abgeleiteten Innenfläche und eine Definition der Zahnersatzbasisgrenzfläche (19) umfasst.

2. Verfahren nach dem vorhergehenden Anspruch, wobei das Bestimmen der Transformation in Schritt iii) derart erfolgt, dass die Transformation die topologische Struktur der Außenfläche in der Zahnersatzaußenfläche (6) erhält.

3. Verfahren nach einem der vorhergehenden Ansprüche, wobei das Bestimmen der Transformation in Schritt iii) derart erfolgt, dass die Transformation die relativen Dicken der Basisschicht, der mindestens einen Übergangsschicht (11) und der Außenschicht (12) in der Zahnersatzbasisschicht, der mindestens einen Zahnersatzübergangsschicht und der Zahnersatzaußenschicht erhält.

4. Verfahren nach einem der vorhergehenden Ansprüche, wobei das Bestimmen der Transformation in Schritt iii) derart erfolgt, dass die Transformation die Lagebeziehungen und Orientierungsbeziehungen der Basisgrenzfläche (13) und der mindestens einen Übergangsschichtgrenzfläche (15) innerhalb eines vorbestimmten relativen Toleranzbereichs in der Zahnersatzbasisgrenzfläche (19) und der mindestens einen Zahnersatzübergangsschichtgrenzfläche erhält.

5. Verfahren nach einem der vorhergehenden Ansprüche, wobei das Bestimmen der Transformation in Schritt iii) mit einem nicht-rigiden Registrierungsverfahren, vorzugsweise mit einem Coherent Point Drift-Algorithmus erfolgt.

6. Verfahren nach dem vorhergehenden Anspruch, wobei der Coherent Point Drift-Algorithmus den geodätischen Abstand zwischen einer Mehrzahl von Punkten auf der Zahnersatzaußenfläche (6) berücksichtigt.

7. Verfahren nach einem der vorhergehenden Ansprüche, wobei in Schritt A) eine Mehrzahl von Schichtmodellen (8) in einer Datenbank bereitgestellt wird, wobei das Verfahren weiterhin den Schritt aufweist
D) Auswählen eines Schichtmodells (8) aus der Mehrzahl von Schichtmodellen in der Datenbank anhand eines für den Zahnersatz zu erzielenden visuellen Gesamteindrucks.

8. Verfahren nach einem der vorhergehenden Ansprüche, wobei das Verfahren zusätzlich die Schritte aufweist
E) Bereitstellen eines Gerüstmodells mit einer Gerüstinnenfläche und einer Gerüstaußenfläche,
wobei die Gerüstaußenfläche von einer Zahnart des zu ersetzenden Zahns abhängt;
F) Erfassen einer Geometrie eines individuellen Zahnstumpfs eines Patienten mit einem bildgebenden Verfahren oder
Definieren einer Außenkontur eines Abutments, welches den Zahnersatz mit einem Kiefer des Patienten verbindet; und
G) Anpassen der Innenfläche des Gerüstmodells, so dass die Innenfläche eine Aufnahme für den Zahnstumpf oder das Abutment ausbildet;
wobei die in Schritt i) festgelegte Zahnersatzinnenfläche (7) oder die aus der Modellinnenfläche abgeleitete Innenfläche die Gerüstaußenfläche ist.

9. Verfahren nach einem der vorhergehenden Ansprüche, wobei das Festlegen der Zahnersatzaußenfläche (6) in Schritt i) unter Berücksichtigung zumindest eines 3D-Scans des zu ersetzenden Zahns oder eines 3D-Scans eines dem zu ersetzenden Zahn benachbarten Zahns erfolgt.

10. Verfahren nach einem der vorhergehenden Ansprüche, wobei das Festlegen der Zahnersatzaußenfläche in Schritt i) durch Auswahl einer geeigneten Zahnform aus einer Datenbank mit anschließender Anpassung an eine individuelle Zahngeometrie des zu ersetzenden Zahns erfolgt.

11. Verfahren nach einem der vorhergehenden Ansprüche, wobei zumindest ein Schichtmodell (8) mindestens eine Übergangsschicht (11) aufweist,
wobei die mindestens eine Übergangsschicht (11) auf der Basisgrenzfläche (13) aufliegt,
wobei die mindestens eine Übergangsschicht (11) einen dritten Parametersatz mit einem dritten Grundfarbton, einer dritten Opazität und einer Übergangsschichtgrenzfläche (15) aufweist,
wobei zumindest der dritte Grundfarbton von dem ersten Grundfarbton verschieden ist oder die dritte Opazität von der ersten Opazität verschieden ist und
wobei in Schritt iv) die Transformation auf die Übergangsschichtgrenzfläche jeder Übergangsschicht angewendet wird, so dass jeweils eine Zahnersatzübergangsschicht berechnet wird.

12. Verfahren nach einem der vorhergehenden Ansprüche, wobei jedes der Schichtmodelle (8) eine Mehrzahl von Übergangsschichten aufweist und wobei die Beschreibung (2) des Zahnersatzes eine Definition einer Mehrzahl von Zahnersatzübergangsschichtgrenzflächen umfasst.

13. Computerprogramm umfassend Befehle, die bei der Ausführung des Computerprogramms durch einen Computer diesen veranlassen, ein Verfahren nach einem der vorhergehenden Ansprüche auszuführen.

14. Verfahren zur Herstellung eines Zahnersatzes mit den Schritten
Bereitstellen der Beschreibung (2) des Zahnersatzes mit einem computerimplementierten Verfahren nach einem der Ansprüche 1 bis 12 und
additives Fertigen zumindest der Basisschicht (10) oder der zumindest einen Übergangsschicht (11) oder der Außenschicht.

15. Datenverarbeitungsvorrichtung mit Mitteln zur Ausführung des Verfahrens nach einem der Ansprüche 1 bis 12 sowie einer Ausgabeeinheit zum Bereitstellen der Beschreibung (2) des Zahnersatzes.
